(19) Europäisches Patentamt
European Patent Office
Office européen des brevets

(11) Publication number: 0 402 226
A1

# (12) EUROPEAN PATENT APPLICATION

(21) Application number: 90401503.9

(22) Date of filing: 05.06.90

(51) Int. Cl.5: C12N 15/62, C12N 15/65, C12N 15/81, C12N 1/16, //(C12N1/16,C12R1:645)

(30) Priority: 06.06.89 EP 89401556

(43) Date of publication of application: 12.12.90 Bulletin 90/50

(84) Designated Contracting States: AT BE CH DE DK ES FR GB GR IT LI LU NL SE

(71) Applicant: INSTITUT NATIONAL DE LA RECHERCHE AGRONOMIQUE
145, rue de l'Université
F-75341 Paris Cédex 07(FR)

(72) Inventor: Fabre, Emmanuel
9 avenue Daniel Lesueur
F-75007 Paris(FR)
Inventor: Nicaud, Jean-Marc
1 rue Florian
F-78190 Trappes(FR)
Inventor: Gaillardin, Claude
7 allée des Gardes Royales
F-78000 Versailles(FR)

(74) Representative: Warcoin, Jacques et al
Cabinet Régimbeau 26, avenue Kléber
F-75116 Paris(FR)

## (54) Transformation vectors for yeast yarrowia.

(57) The present invention relates to a hybrid DNA sequence comprising a new metabolic gene and preferably the SUC2 gene, under the control of a promoter and signal sequence functional in a Yarrowia strain.

The present invention concerns too the process of obtaining these Yarrowia strains transformed with a hybrid DNA sequence.

EP 0 402 226 A1

The present invention deals with the transformation of Yarrowia with hybrid DNA sequences carrying heterologous genes coding for metabolic activities new to Yarrowia (hereinafter "new metabolic genes") and with uses of said new metabolic genes.

Transformation of the industrial yeast Y. lipolytica has been set up using the S. cerevisiae LYS2 gene (Gaillardin et al. 1985) and the Y. lipolytica LEU2 gene (Davidow et al. 1985). Since then, other biosynthetic genes such as BIO, URA3, HIS1 (Davidow et al. 1987), LYS5 (Xuan et al. 1988) have been cloned and isolated. These cloned genes could be used as selective markers into suitable recipients. In all cases studied, transformation resulted from integration of the vector(s) into the chromosome of the host.

While the number of selective markers available is increasing, there is a need for universal markers for transformation. Indeed, dominant selective marker are useful for the transformation of strains where induction of auxotrophic mutations is either difficult or undesirable. It could also be important if isogenic strains are desired or to avoid to recover conversions and reversions. Y. lipolytica was unfortunately found to be resistant to most antibiotics commonly used in S. cerevisiae, including chloramphenicol or G418 (Cohen et al. 1980 ; Jimenez and Davies 1980). The inventors have reported the development of a visual marker using the lacZ gene of E. coli expressed under the LEU2 promoter which produces blue colonies in the presence of Xgal as well as the expression of a gene conferring phleomycin resistance in Y. lipolytica - (Gaillardin and Ribet 1987). Direct selection of Phleo$^R$ transformants was possible on 15 mg.l phleomycin. However, using this concentration at least half of the Phleo$^R$ clones obtained resulted form spontaneous Phleo$^R$ mutations in the recipient strains. The frequency of these false transformants was decreased by using an expression phase before plating. The draw back was a 60 fold reduction in the transformation frequency. Moreover, it remains the possibility of inducing mutations, since phleomycin induces double strand breaks in the DNA (Hecht 1986). So, the purpose of the present invention is the development of another type of selection.

Y. lipolytica is a dimorphic yeast which can grow on a limited number of carbon sources, including glucose and glycerol, paraffines such as n-alkanes and alkenes (Klug and Markovetz 1967, 1969 ; Bassel and Mortimer 1973), lipids and proteins (Ogrydziak et al. 1977) but not sucrose. To degrade proteins, depending on growth conditions, Y. lipolytica secretes proteases (Ahear et al. 1968, Kamada et al. 1972). For example, in alkaline medium, it secretes an Alkaline Extracellular Protease (AEP) (Tobe et al. 1976; Ogrydziak et al. 1977, Ogrydziak and Schaf 1982). The corresponding gene, XPR2, has been cloned independently by Davidow et al. (1987), Matoba et al. (1988) and in our laboratory.

In this patent, it its described the expression and secretion in Yarrowia especially Y.lipolytica of new metabolic genes, especially of the S. cerevisiae SUC2 gene under the control of a promoter especially the promoter and signal sequences of the XPR2 gene.

The expression of the invertase activity confers a sucrose utilizing (Suc$^+$) phenotype. Invertase activity follows the same regulation as AEP activity and is secreted into the periplasm with only about 10 % secreted into the culture medium. the chimeric invertase gene was used as a dominant marker for transformation in a one step procedure.

This is a reason why in the present invention the hybrid DNA sequence comprising the SUC2 gene is preferably under the control of a promoter and signal sequence functional in Yarrowia especially in a Y. lipolytica strain.

Although different promoters and signal sequences may be used the promoter and sequences prefered are those with which the expression product of SUC2 gene is expressed mainly in the periplasm of Yarrowia if necessary under specific culture conditions. For example, the use of promoter and signal sequence of XPR2 is especially appropriated. It is to be noted, however, that use of the pre and prepro sequence of XPR2 results in secretion of gene products into the medium (Franka et al. 1988 -Developments in Industrial Microbiology 29, 43-57). Said hybrid DNA sequence may be used as a dominant marker for several uses.

Said hybrid DNA sequence may be used for integration using homologous recombination. In this case, the hybrid DNA sequence must be flanked by DNA regions homologous to a DNA region present in the genome of the recipient Yarrowia strain.

Said hybrid DNA sequence may be used also on a plasmid comprising a ARS region for autonomous replication. Said ARS may be of genomic origin or from a plasmidic origin. In this case, the marker may be used to maintain the plasmid without using an antibiotic pressure in the culture medium.

The XPR2 promoter and signal sequence could be used to target periplasmic secretion in Y. lipolytica of the S. cerevisiae invertase encoded by the SUC2 gene.

Invertase production in Y. lipolytica strains transformed by the chimeric plasmid is sufficient to allow growth on sucrose although Y. lipolytica does not grow on sucrose as a sole carbon.

As in S. cerevisiae, invertase activity remained mainly in the periplasm and cell wall with only 10 % secreted into the culture medium. Invertase production follows the same regulation as the AEP (Tobe et al.

1976 ; Ogrydziak et al. 1977 ; Ogrydziak and Scharf 1982). Since secretion of very little invertase activity is sufficient to allow growth on sucrose (Robinson et al. 1988), absence of growth of transformants in YNBSa indicates a very tight regulation of the promoter. AEP activity in YNB medium is not observed. Little expression was observed in YNBP, and a 200 fold amplification is obtained in YPDm confirming that the XPR2 promoter is a strong and well regulated promoter.

Therefore the SUC2 gene, as an example of a dominant screenable marker, can be used in Y.lipolytica and this marker presents several advantages :

- At most a 2 fold reduction of the transformation frequency with $Leu^+$ vs. $Suc^+$ selection, while with the phleomycin resistance selection a 60 fold reduction of the transformation frequency was observed (Gaillardin and Ribet 1987).
- All $Suc^+$ strains are transformants since no invertase gene is present in Y.lipolytica and we never observed appearance of spontaneous $Suc^+$ clones. We thus could eliminate the problems due to spontaneous resistance which obscured the phleomycin selection, but also those due to conversion events, reversion or leakiness of mutation potentially associated with conventional auxotrophic markers.

Said invention using the invertase fusion or other new metabolic gene could be used in any prototrophic or auxotrophic Y. lipolytica strains as a dominant marker for transformation. Such a fusion could also be used as dominant marker for genetic mapping experiments or gene disruption : for example isogenic $URA3^+/ura3^-$ strains could be obtained by gene replacement with an ura3 : XPR2-SUC2 disrupting vector at the wild type $URA3^+$ locus. And finally production of a new metabolic gene product such as invertase by these strains allows utilization of a new substrate by the industrial yeast Y. lipolytica , especially sucrose and some material containing sucrose such as black strap molasses and beet molasses.

The use of Yarrowia lipolytica for expression of foreign proteins has been disclosed in detail in the previous patents and the new metabolic genes of the present invention may be used in the construction described previously.

The following examples will help to understand other advantages and characteristics of the invention.

Fig. 1: A,B. Sequence of the upstream region of the XPR2 gene.

A: Nucleotide sequence and amino acid translation of the 553bp XhoII- BglII fragment of the Y. lipolytica XPR2 gene cloned in phage M13mp18 (M13mp18-X4). Part of the M13mp18 multisite is underlined. The BamHI site which has been recreated in 5′ and the 3′ SalI site were used for the construction of pINA165. The position of the EcoRV site used in the construction of pINA150 and pINA166 (see Fig. 2) is indicated. Perfect matches with the mutagenic primer used to introduce a HindIII site at the signal sequence cleavage site have been indicated by (*) above the sequence.

B: Nucleotide sequence and amino acid translation of the surrounding region of the putative signal sequence cleavage site (indicated by an arrow in A) and the corresponding mutated sequence. Above is indicated the fusion site with the SUC2 gene.

Fig. 2: construction of the chimeric plasmid (pINA169) for the expression of the S. cerevisiae SUC2 gene in Y. lipolytica. A: (—) pBR322 DNA, ( ▼▼ ) Y. lipolytica XPR2 promoter from pINA105, (●●) LEU2 sequence from pINA62 and the SUC2 gene from pRB58. B: schematic map of the phage M13mp18-X4 and its mutagenized derivative M13mp18-X4m presenting the new PstI and HindIII restriction sites.

Fig. 3: Restriction map of the plasmid pINA169 carrying the chimeric fusion. Symbols are; ( ▇ ) pBR322, ( ▒ ) XPR2, ( ▨ ) LEU2 and ( ▩ ) SUC2 sequences.

Fig. 4: Production of invertase activity by Y. lipolytica strain JM58 carrying the SUC2 gene integrated at the XPR2 locus. Strain JM58 was grown at 28 °C in YNBP S ( ■/□ ), YPDmG ( ●/○ ) and YPDmS ( ▲/△ ). Growth is indicated as full symbols and activity as empty symbols.

Fig. 5: Construction of plasmid pINA 302.

The hybrid DNA sequence composed of the SUC2 gene under the control of the XPR2 promoter and signal sequence was isolated on a NruI-Sal fragment and inserted in the URA3 gene between the EcoR V and XhoI sites.

Fig. 6: Construction of plasmid pINA270 and pINA322.

## MATERIALS AND METHODS

### Strains and plasmids

Y. lipolytica strain JM12 used was derived form a cross between 21501-4 (Xuan et al 1988) and W29 ura3-18 (our laboratory) as being phenotypically $Ura3^-$, $Lys5^-$, $Leu2^-$, $Xpr^+$ and highly transformable with pINA62. Y. lipolytica strains were constructed using standard genetic techniques (Gaillardin et al. 1973), genetic markers ade1 and leu2-35 derive from D. Ogrydziak's collection (Ogrydziak et al. 1982) and form ATC 20688. The strain JM23 with no active copy of the XPR2 gene derives from JM 12 and was constructed by gene disruption, using an XPR2 : Lys 5 vector (our laboratory).

The Y. lipolytica XPR2 gene was isolated from partial genomic libraries of Y. lipolytica DNA in pBR322 using oligonucleotide probes based on the N-terminal amino acid sequence of mature Alkaline Extracellular Protease

Plasmid pINA152 carries the complete XPR2 gene on a 4,2Kb PstI fragment, whereas pINA105 contains only the 5′ half of the coding sequence including promoter and presequences.

Escherichia coli strain HB101 was used for plasmid construction and propagation and TG1 for phage preparations.

Media. The following media were used. YNB minimal medium is described in Sherman et al. (1979), YNBG and YNBS refer to this medium with glucose or sucrose (respectively) as carbon sources. YNBGPo, YNBSaPo , YNBSfPo are YNB media buffered at pH6.8 with 50mM phosphate buffer, the carbon source being glucose (G), or autoclaved/filtered saccharose (Sa/Sf). Phosphate buffer (Po) and proteose peptone (1.7g/l, $P_P$) were added to induce the AEP promoter. Minimal media were supplemented as required with amino acids and bases 50mg/l except for leucine (200mg/l). YPDm correspond to the YPD medium (Gaillardin and Ribet 1987) with the following modifications: yeast extract (lg/l) and proteose peptone (50g/l). YPDmG and YPDmS correspond to YPDm with glucose 10g/l or saccharose 10g/l as carbon source, respectively.

E. coli media were LB and M9 prepared as described in Miller (1972). Recombinant M13 phage were grown on 2X TY medium (1.6% Difco Bactotryptone, 1% yeast extract, 0.5% NaCl).

DNA transformation. E. coli were transformed using the $CaCl_2$-method described by Maniatis et al. (1982) or the method of Dagert and Ehrlich (1979).

High frequency Y. lipolytica transformation was done essentially as described by Gaillardin et al. (1985), Xuan et al. (1988). Rapid transformation of Y. lipolytica was performed as follows: two loops of fresh growing cells from YMC plates [ yeast extract (3g/l), proteose peptone (5g/l), malt extract (3g/l) and sodium citrate (0.2g/l)] were resuspended in 1 ml TE (Tris 10mM, EDTA 1mM pH8) in an 1.8ml eppendorf tube. Cells were spun down and resuspended in 600ul of 0.1M lithium acetate pH6 (LiAC). After one hour incubation at 28°C, the cells were spun down and resuspended in 60ul of the same buffer. To 20ul of competent cells, we added 2.5ul of carrier DNA and 1.5ul of plasmid to be transformed (about 0.1ug). After incubation at 28°C for 15 min., 300ul of PEG solution (PEG 40% in LiAC buffer) was added. After incubation at 28°C for one hour and at 39°C for 10 min. (heat shock), 600ul of lithium buffer were added. Then, 100ul of the cells were plated on selective media.

DNA preparation. Plasmid DNA was prepared by the Holmes and Quigley (1981) procedure. DNA fragments were separated by electrophoresis on agarose and purified using a Gene Clean kit (OZYME) according to the manufacturer.

Oligonucleotide mutagenesis. A 553 bp XhoII-BglII fragment of pINA105 spanning the 5′ upstream region of XPR2 (see fig. 1A) was inserted into the replicative form of M13mp18 digested by BamHI, the two orientations were obtained M13mp18-X4 and M13p18-X8. A 26 mere (5′-CAGCATCAGAAGCTTCTGCAGGGGCG-3′) oligonucleotide was kindly synthesized by B. Dujon using the solid phase phosphotriester method (Efimov et al. 1983). Oligonucleotide mutagenesis was performed according to Eckstein's method (Taylor et al. 1985) using Amersham's kit. Mutagenized phage samples, grown from plaques selected at random, were applied to a nitrocellulose filter (Millipore) and probed with the 5′ end $^{32}P$ labelled mutagenic oligonucleotide. The filter was then exposed for autoradiography. Positive inserts were checked by DNA sequencing.

Enzymes. Restriction enzymes, T4 DNA ligase, and DNA-polymerase I (Klenow fragment) were purchased from Boehringer Mannheim and polynucleotide kinase from Amersham. The enzymes were used according to manufacturer's specifications.

DNA sequencing. DNA sequencing was performed according to the Sanger dideoxy method using M13mp18 and M13mp19 phages (Yanisch-Perron et al. 1985) as described in Sanger et al. (1977). $^{32}P$-dCTP was used for labeling.

Invertase essay. Invertase activity was determined according to Werner et al. (1970) using the test combination glucose from Boehringer Mannheim. Culture samples were taken during growth and cells recovered by centrifugation were resuspended in the assay buffer. Activity was determined in the supernatant and on whole cells(no difference was observed between whole cells and broken ones). One unit of enzyme is defined as the amount of enzyme which produces an OD increase of 1 at 610 nm per min. at 45°C.

EXAMPLE 1 :

Construction of pINA169 (see fig. 2)

The S. cerevisiae SUC2 gene was isolated from the plasmid pRB 58 (Carlson and Botstein, 1982) and the Y. lipolytica LEU2 gene was isolated from plasmid pINA62 (Gaillardin and Ribet, 1987). Plasmid pINA 105 was the source of the XPR2 promotor and M13mp18-X4m phage (see Fig. 1) for the 5' part of the XPR2 gene and the signal sequence with an HindIII site for the fusion with the SUC2 gene. This site is located after the more likely site for signal sequence cleavage site which is after $Proline_1$ as determined with the method described by von Heijne (1986). This mutagenized phage was obtained as described in Materials and Methods. This restriction site should generate a XPR2 signal sequence::SUC2 in frame fusion as described in Fig.1,B. This fusion puts the 23 N-terminal amino acids from the XPR2 in front of invertase starting at amino acid eleven (see Fig.1B).

The main part of the XPR2 promoter located on a 1,88Kb HindIII-EcoRV fragment isolated from pINA 105 was inserted in pBR322 (pINA150).

The pBR322 HindIII site was eliminated by a ClaI deletion (pINA151). The 565 bp fragment carrying the new restriction site in the signal sequence in M13mp18-X4m (see oligonucleotide directed mutagenesis and Fig.1) was gel purified and inserted in the SalI-BamHI fragment from pINA151 to generate plasmid pINA165. By deletion of the EcoRV fragment in pINA165 we generated plasmid pINA166 which carries the XPR2 promoter and the signal sequence followed by an HindIII restriction site. Plasmid pINA167 resulted from the insertion of the 5,6Kb SalI fragment carrying LEU2 from pINA62 (Gaillardin and Ribet 1987) into pINA166. The orientation of LEU2 was checked by EcoRI digestion. Then the 2Kb HindIII fragment carrying the SUC2 gene deleted of the ATG and part of the signal sequence (the HindIII site is located at position 11 from the ATG; Taussig and Carlson 1983) was inserted downstream from the XPP2 promotor to generate plasmid pINA169. The desired orientation of the insertion was checked by BamHI-BglII digestion (see Fig. 3).

EXAMPLE 2 :

EXPRESSION OF THE INVERTASE FUSION IN Y.LIPOLYTICA

Y. lipolytica strain JM23, (see Table 1) which is a strain with a disruptedXPR2 gene was transformed either with the chimeric plasmid pINA169 or with pINA62 as a control plasmid. In order to increase the transformation frequency, plasmids were linearized by digestion with a restriction enzyme prior to the transformation experiment. Plasmid pINA169 was targeted to the XPR2 locus by cutting the plasmid at the unique NheI site located in the XPR2 promoter region or at the BglII site located in the LEU2 sequence (fig.3). Plasmid pINA62 was linearized by ApaI within the LEU2 sequence (Fig.3). With both plasmids, $Leu^+$ transformants were obtained at high rate, about 30 000 transformants per µg of DNA (see also Table 3). However, differences were observed depending on the site of, integration. Indeed, using a rapid transformation procedure, by NheI restriction integration occurred at the XPR2 locus at a frequency of 700 transformants/ug whereas by BglII restriction integration occured at the LEU2 locus at a frequency of 1943 transformants/µg. This difference could reflect the length of homology shared by the plasmid and the target locus: there are 0.49 and 0.93Kb of homology with the XPR2 locus versus 2.6 and 2.5Kb with the LEU2 locus. Several hundred transformants obtained with each plasmid were streaked on YNB and tested for $Suc^+$ phenotype on YNBSa and $YNBSaP_0$ plates. No pINA62 transformants were able to grow on saccharose. All pINA169 transformants were $Suc^+$ on $YNBSP_O$ , whereas none were $Suc^+$ on YNBS after one week incubation. No differences were observed for the $Suc^+$ phenotype depending on the site of integration (XPR2 or LEU2 locus). This result indicates that the SUC2 gene of S. cerevisiae confers a $Suc^+$ phenotype to Y. lipolytica. This phenotype was due to the expression of the SUC2 gene which followed the same regulation as the AEP (see also the following section). Indeed, no expression of AEP was observed in YNB which parallels the absence of growth of transformants on YNBS. However, after two weeks of incubation of the pINA169 transformants on YNBS, single colonies arose form the background.

**EXAMPLE 3**

## Production and secretion of invertase activity in pINA169 transformants

To define media and conditions were the Suc$^+$ phenotype could be selected for directly after transformation, the medium must meet the following conditions : the invertase production must be sufficient to permit growth, the level of extracellular invertase must be limiting to prevent crossfeeding and the Suc$^-$ strains must not grow. Ogrydziak and collaborators (Ogrydziak et al. 1977, Ogrydziak and Scharf 1982) reported about the regulation of protease production by carbon, nitrogen, sulfur sources and pH. Invertase production have been measured during growth in several media in the presence of glucose or saccharose as carbon sources. In each medium about 10% of the activity was observed in the culture broth whereas 90% of the activity could be recovered using whole cells. Invertase activities found in the media tested are summarized in Table 2.

The plasmid pINA 169 has been used to transform an E. coli strain which has been referenced as E. coli INA G 20765.

Table 2

| Comparison of invertase production by Suc$^+$ transformants depending on growth medium. Invertase activity was measured in stationary phase and is expressed in unit per ml of culture. | |
|---|---|
| MEDIUM | INVERTASE ACTIVITY |
| YNBS | no growth |
| YNBP.G | 0.027 |
| YNBP.S | 0.02 |
| YNBP.$P_P$G | 0.4 |
| YPD | 0.17 |
| YPDmG | 0.8-1.1 |
| YPDmS | 0.8-1.3 |

No detectable invertase activity was observed in non buffered YNB with glucose or saccharose as a carbon source. Various induction factors were obtained for invertase in pH 6.8 buffered YNB or on YPL media. Fig.4 shows that maximum derepression occurred at the end of the exponential phase and that the kinetics of production is dependent of the medium. As shown in Table 2, medium YNBP presented the desired properties for direct selection. We have therefore used this medium to test for the direct selection of transformants based on their Suc$^+$ phenotype.

EXAMPLE 4 :

## Selection of transformants with Suc$^+$ phenotype.

To test if the Suc$^+$ phenotype could be used as a selective marker, we transformed strain JM23 with pINA169 cut by NheI. Since very little invertase activity was observed in the supernatant of cells grown in YNBP$_\bullet$ , it seemed likely that this level would not be sufficient for efficient cross-feeding of the Suc$^-$ recipient strain on saccharose and that it should be possible to screen transformants on selective medium directly. However we thought that small amounts of glucose could be necessary to facilitate recovery of the transformants and production of invertase.

Y. lipolytica strain JM23 was transformed using the rapid procedure (see Material and Methods) and cells were plated on YNB Ura (selection of transformants using the complementation of the auxotrophic mutation leu2) and on YNBSfP• or YNBSaP• supplemented with uracil and leucine (selection of transformants using $Suc^+$ phenotype). As shown in Table 3, we obtained 630 transformants per ug of DNA using the LEU2 selection, no transformants on YNBSfP. and 350 transformants per ug of DNA on YNBSaP . The difference between these two media confirmed our hypothesis that small amounts of glucose produced by thermal hydrolysis of saccharose during autoclaving was necessary and sufficient for the recovery of the transformants and the production of invertase. We have tested successfully this selection on several strains. For example, using the high frequency transformation procedure (Gaillardin et al. 1985) we have transformed strains 11104 and obtained 33150 transformants per ug of DNA using the $Leu^+$ selection and 24000 transformants using the $Suc^+$ selection (see Table 3). Selecting for $Leu^+$, transformants gave colonies within two to three days while upon $Suc^+$ selection, transformants appeared within 4 to 5 days

Genetic analysis by Southern blotting confirmed in each case that a complete plasmid was inserted at the XPR2 locus.

**Table 3: Comparison of the transformation efficiency of Y. lipolytica strains by plasmid pINA169 linearized by NheI when selection was applied either for complementation of the auxotrophic marker leu2-35 or for growth on sucrose plates. The two transformation procedures used are described in materials and methods.**

| TRANSFORMATION METHOD | STRAINS | MEDIA | SELECTION | TRANSFORMATION |
|---|---|---|---|---|
| Rapid transformation | JM23 | YNBP.ura G | $Leu^+$ | 630 trf./ug |
| | JM23 | YNBP.uraleu Sa | $Suc^+$ | 350 trf./ug |
| | JM23 | YNBP.uraleu Sf | $Suc^+$ | 0 trf./ug |
| high frequency transformation | 11104 | YNBP. G | $Leu^+$ | 33150 trf./ug |
| | 11104 | YNBP.leu S | $Suc^+$ | 24000 trf./ug |

**EXAMPLE 5 :**

## Gene disruption of URA3

The URA3 gene of Y. lipolytica has been cloned on a Sau3A fragment of 4,2 kb and sub cloned on a SalI fragment in vector pUC13.

This vector is digested by XhoI and EcoRV and the fragments containing URA3 have deplaced by NruI-SalI fragment of 3,3 kb of pINA169 containing the fused gene XPR2-SUC2. The resulting plasmid pINA302 (see fig. 5) contains the fused gene XPR2-SUC2 insert between 570 bp in 3′ of URA3 et 400 kp of 5′ of URA3. The disrupting cassette may be excised of pINA302 by SalI and can be used for disrupting URA3.

Strain AHA (mat A, his1) has been transformed by pINA 302 digested by SalI (targeting URA3 and disruption of locus). The transformants have been selected on YNB Po sucrose, histidine, uracil. 10-50 transformants/µg were obtained. These last ones were sub cloned in isolated colonies and tested for uracil requirement. 30 % were $ura^-$ and the disruption has been checked by Southern blot.

The plasmid pINA 302 has been used to transform an E. coli strain which has been referenced as E.

coli INA G. 20766.

## EXAMPLE 6

### Gene replacement using URA3::SUC2 disruption

URA3::SUC2 disruptions display a non reverling, non leaky $ura^-$ phenotype. This was used to manipulate industrial strains which otherwise could not be easily dealt with, unless markers and possibly secondary unwanted mutations are introduced by standard mutagenesis.

The industrial strains W29 (ATCC 20460) was transformed with pINA302 digested by SalI and SUC $ura3^-$ transformants were isolated as before; one was saved and called Po. We wanted to generate a $leu2^-$ marker for further transformation assays and to destroy the XPR2 gene which encodes the major alkaline extracellular protease (AEP). We constructed pINA270 (see Fig. 6) which carries a complete URA3 gene and a deleted LEU2 gene. This was obtained by digesting pINA62 (Gaillardin and Ribet, 1987) with StuI and religating the plasmid on itself, followed by insertion of URA3. Similarly, we constructed pINA322 (see Fig. 6) which carries URA3 and a deleted XPR2 gene. This was obtained by removing from the XPR2 sequence the ApaI fragment encompassing transcription and translation starts as well as part of the preprosequence of AEP. Strain Po ($ura3^-$) was transformed to URA′ with pINA270 cut by NoLI. Transformants carrying the plasmid integrated at LEU2 were plated ($10^6$ cells per plate) on YNB containing 15 mg/l uracile, 100 mg/l leucine and 1.25 g/l fluorooprotic acid (5FOA). 5FOA is converted by the product of the URA3 gene into 5-fluoro-UMP which is toxic to the cell. ura3 $5FOA^R$ clones appeared at a frequence of $10^{-4}$ cell plated and were shown by Southern to result from reexcision of the integrated plasmid (Boecke et al. 1984). About half of the $5FOA^R$ clones were simultaneously $leu^-$ and carried the StuI deleted LEU2 gene (Southern blot, not shown). One strain was saved as Pola. It was then transformed by pINA322 cut by MluI and $URA^+$ transformants were selected. The reexcision of the plasmid was selected as before on YNB + 5FOA + uracile + leucine, and $Aep^-$ derivatives were isolated. They were shown by Southern to carry both LEU2 and XPR2 deletions. One strain was saved as Pold. It was then retransformed with the SalI fragment of pINA156 carrying URA3 and URA′ $Suc^-$ clones were obtained. The resulting strain Poiu is isogenic to the starting strain W29 except for the LEU2 and XPR2 deletions and does not carry any foreign sequence.

Pold and a laboratory strain carrying UV induced leu2 and xpr2 mutations (Fournier P. et al. 1989) were transformed with a replicative plasmid carrrying the LEU2 gene and a AEP:porcine alpha interféron gene fusion (Fournier P. et al. 1989). Antiviral activity was measured using a cytopathy test and IFN protein was quantified using an ELISA test. Results shown on table 4 show that the industrial strain was indeed superior to the laboratory strain.

Table 4 :

| JM23 | | Pold | |
|---|---|---|---|
| antiviral | elisa | antiviral | elisa |
| 130 | 135 | 400 | 600 |

The strains E. coli INA G 20765 and E. coli INA G 20766 have been filed on June 5. 1989 under the designations I 865 and I 866 at the "National Collection of Microorganisms" of the INSTITUT PASTEUR in Paris (France).

Ahearn DG, Meyers SP, Nichols RA (1968) Appl Microbiol 16:1370-1374

Bassel J, Mortimer R (1973) J Bacteriol 114:894-896

Boecke J.D., Lacroute F., Fink G.R. (1984) Mol. Gen. Genet. 197, 345-346

Boyer HW, Roulland-Dussoix D (1969) J Mol Biol 41:459-472

Carlson M, Botstein D (1982) Cell 28:145-154

Cohen JD, Eccleshall TR, Needlemann RB, Federoff H, Buchferer BA, Marmur J.(1980) Proc Natl Acad Sci USA 77:1078-1082

Dagert M, Ehrlich SD (1979) Gene 6:23-28

Davidow LS, Apostolakos D, O'Donnell MM, Proctor AR (1985) Curr Genet 10:39-48

Davidow LA, Franke AE, DeZeeuw JR (1987) European Patent Application 220864
Davidow LS, O'Donnell MM, Kaczmarek FS, Pereira DA, De Zeeuw JR, Franke AE (1987) J Bacteriol 169:4621-4629
Efimov VA, Burykova AA, Reverdato SV, Chakhmakhcheva CG, Ovchinnikov YA (1983) Nucl Acid Res 11:8369
Fournier P. et al. (1989), EP-O 329 501
Gaillardin CM, Charoy V, Heslot H (1973) Arch Microbiol 92:69-83
Gaillardin C, Ribet AM, Heslot H (1985) Curr Genet 10:49-58
Gaillardin C, Ribet AM (1987) Curr Genet 11:369-375
Hecht SM (1986) Federation Proc 452784-2791
Holmes DS, Quigley M (1981) Anal Biochem 114:193
Jimenez A, Davies J (1980) Nature 287:869-871
Kamada M, Ogura S, Oda K, Murao S (1972) Agric Biol Chem 36:171-175
Klug MJ, Markovetz AJ (1967) J Bacteriol 93:1847-1851
Klug MJ, Markovetz AJ (1969) Biotech Bioeng 11:427-440
Maniatis T, Fritsch EF, Sambrook J (1982) Molecular cloning. Cold Spring Harbor Laboratory, Cold Spring Harbor, NY
Miller JH (1972) Experiments in molecular genetics. Cold Sprang Harbor Laboratory, Cold Spring Harbor, NY
Ogrydziak DM, Demain AL, Tannenbaum SR (1977) Biochem Biophys Acta 497:525-538
Ogrydziak DM, Scharf SJ (1982) J Gen Microbiol 128:1225-1234
Robinson JS, Klionsky DJ, Banta LM, EMR SD (1988) Mol Cell Biol 8:4936-4948
Sanger F, Nicklen S, Couloon AR (1977) Proc Nat Acad Sci USA 74:5463-5467
Sherman F, Fink GR, Hicks JB (1979) Methods in yeast genetics. Cold Spring Harbor Laboratory, Cold Spring Harbor, NY, pp90-92.
Sreekrishna K, Tschopp JF, Fuke M (1987) Gene 59:115-125
Taussig R, Carlson M (1983) Nucl Acid Res 11:1943-1954
Taylor JW, Ott J, Eckstein F (1985) Nucl Acid Res 13:8764-8785
Tobe ST, Takami S, Ikeda S, Mitsugi K (1976) Agr Biol Chem 40:1037-1092
von Heijne G (1986) Nucleic Acids Res 14:4683-4690
Werner W, Rey HG, Wielinger H (1970) Z Analyt Chem 252:224-225
Xuan J-W, Fournier P, Gaillardin C (1988) Curr Genet 14:15-21
Yanisch-Perron C, Vigra J, Messing J (1985) Gene 33:103-119

## Claims

1. A hybrid DNA sequence comprising a new metabolic gene under the control of a promoter and signal sequence functional in a Yarrowia strain.

2. A hybrid DNA sequence according to claim 1 wherein the new metabolic gene is theSUC2 gene.

3. A hybrid DNA sequence according to claim 1 wherein the promoter and signal sequence control the expression of the new metabolic gene such that the product of said gene is located mainly in the periplasm of a Yarrowia strain.

4. A hybrid DNA sequence according to claim 2 wherein the promoter and signal sequence control the expression of the SUC2 gene such that the product of said gene is located mainly in the periplasm of a Yarrowia strain.

5. A hybrid DNA sequence according to claims 1 to 4 wherein the promoter is the XPR2 promoter and the signal sequence is the XPR2 signal sequence.

6. A hybrid DNA sequence according to claims 1 to 5 wherein the Yarrowia strain is Yarrowia lipolytica.

7. A hybrid DNA sequence according to claims 1 to 6, flanked by DNA sequences homologous to a DNA region present in the genome of the Yarrowia strain.

8. A hybrid DNA sequence according to claims 1 to 7 wherein said sequence is within a plasmid.

9. A hybrid DNA sequence according to claim 8 wherein the plasmid comprises an ARS region effective in the Yarrowia strain.

10. A Yarrowia strain transformed with a hybrid DNA sequence according to claims 1 to 9.

11. Use of a hybrid DNA sequence as described in claims 1 to 9 as a dominant marker for transformation of a prototrophic or auxotrophic Yarrowia strain.

12. A process of obtaining a Yarrowia strain capable of utilizing sucrose for growth which comprises transforming said Yarrowia strain with a hybrid DNA sequence according to claims 1 to 9 wherein the new metabolic gene is the SUC2 gene.

13. A process for obtaining transformants of a Yarrowia strain which comprises transforming cells of said Yarrowia strain with a hybrid DNA sequence according to claims 1 to 9 and cultivating the resulting transformed cells under conditions requiring the functioning of the metabolic gene of said hybrid DNA sequence for growth of said cells.

14. A process according to claim 13 wherein the Yarrowia strain is Y. lipolytica.

15. Plasmid pINA169.

16. Plasmid pINA302.

17. A process for disrupting a gene of a Yarrowia strain which comprises transforming cells of said Yarrowia strain with a hybrid DNA sequence according to claim 7.

18. A process according to claim 16 wherein the hybrid DNA sequence is within a plasmid.

19. A process according to claim 17 or 18 wherein the Yarrowia strain is Y.lipolytica.

A

BamHI(BamHI/XhoII)
CCCGGGgatcccactact -360

tgtagtcaggccatcttttacgtacgcactgtaccatgatgtcaatggagtatgatgaaccgactttgagagactcacatctgcacaacaccatgtttcagcggaatccgactttccaac -240

EcoRV
ccaaacccaagcccctgtcagatatcgtgagaaggcacggcaccaactaatgcacacactccacctgtattgcaccaagataatgacgggcatcgtcttggcgcgtcttggcgagagccg -120

tgtttcgtgacgcaatcagagcagtttctggatagtatcttgtccagaacacgatataaaccccatcgacgggcccgttgaagagcaccaacccactatccaatcctccaatccaaca -1

* *** *** ** *** ** *** *** *

ATG AAG CTC GCT ACC GCC TTT ACT ATT CTC ACT GCC GTT CTG GCC GCT CCC CTG GCC GCC CCT GCC CCT GCT CCT GAT GCT GCC CCT GCT 90
Met Lys Leu Ala Thr Ala Phe Thr Ile Leu Thr Ala Val Leu Ala Ala Pro Leu Ala Ala Pro Ala Pro Ala Pro Asp Ala Ala Pro Ala 30
-X-Ala, -X-Pro, -X-Gly dipeptides I

ApaI
GCT GTG CCT GAG GGC CCT GCC GCC GCT GCC TAC TCA TCT ATT CTG TCC GTG GTC GCT AAG CAG TCC AAG AAG TTT AAG CAC CAC AAG TGA 180
Ala Val Pro Glu Gly Pro Ala Ala Ala Ala Tyr Ser Ser Ile Leu Ser Val Val Ala Lys Gln Ser Lys Lys Phe Lys His His Lys Arg 50

BglII/BamHI
GAT CCTCTAGAGTCGAC--
Asp XbaI SalI

B

11 12 13
Ala Phe Leu
AA GCT TTC CTT --- SUC2
HindIII

M13mp18-X4a C GCC CCT GCA GAA GCT TCT GAT GCT G
M13mp18-X4 C GCC CCT GCC CCT GCT CCT GAT GCT GCC
XPR2 Ala Pro Ala Pro Ala Pro Asp Ala Ala
20 21

FIG_1

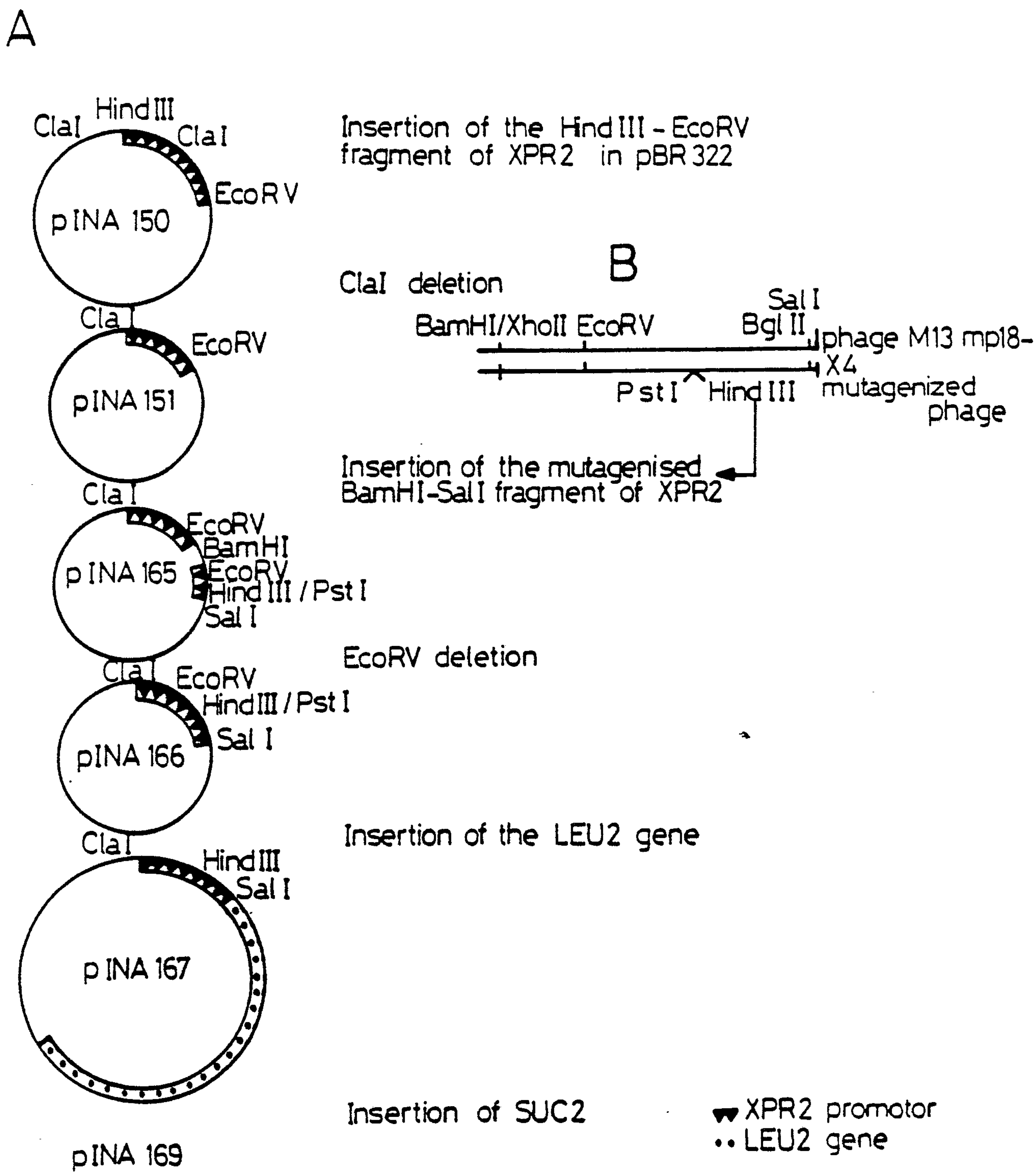
A
ClaI
Hind III
ClaI
EcoRV
pINA 150
Insertion of the HindIII - EcoRV fragment of XPR2 in pBR322
ClaI deletion
ClaI
EcoRV
pINA 151
B
SalI
BamHI/XhoII EcoRV
Bgl II
phage M13 mp18-X4
PstI
HindIII
mutagenized phage
Insertion of the mutagenised BamHI-SalI fragment of XPR2
ClaI
EcoRV
BamHI
EcoRV
HindIII / PstI
SalI
pINA 165
EcoRV deletion
ClaI
EcoRV
HindIII / PstI
SalI
pINA 166
Insertion of the LEU2 gene
ClaI
HindIII
SalI
pINA 167
Insertion of SUC2
XPR2 promotor
LEU2 gene
pINA 169

FIG_2

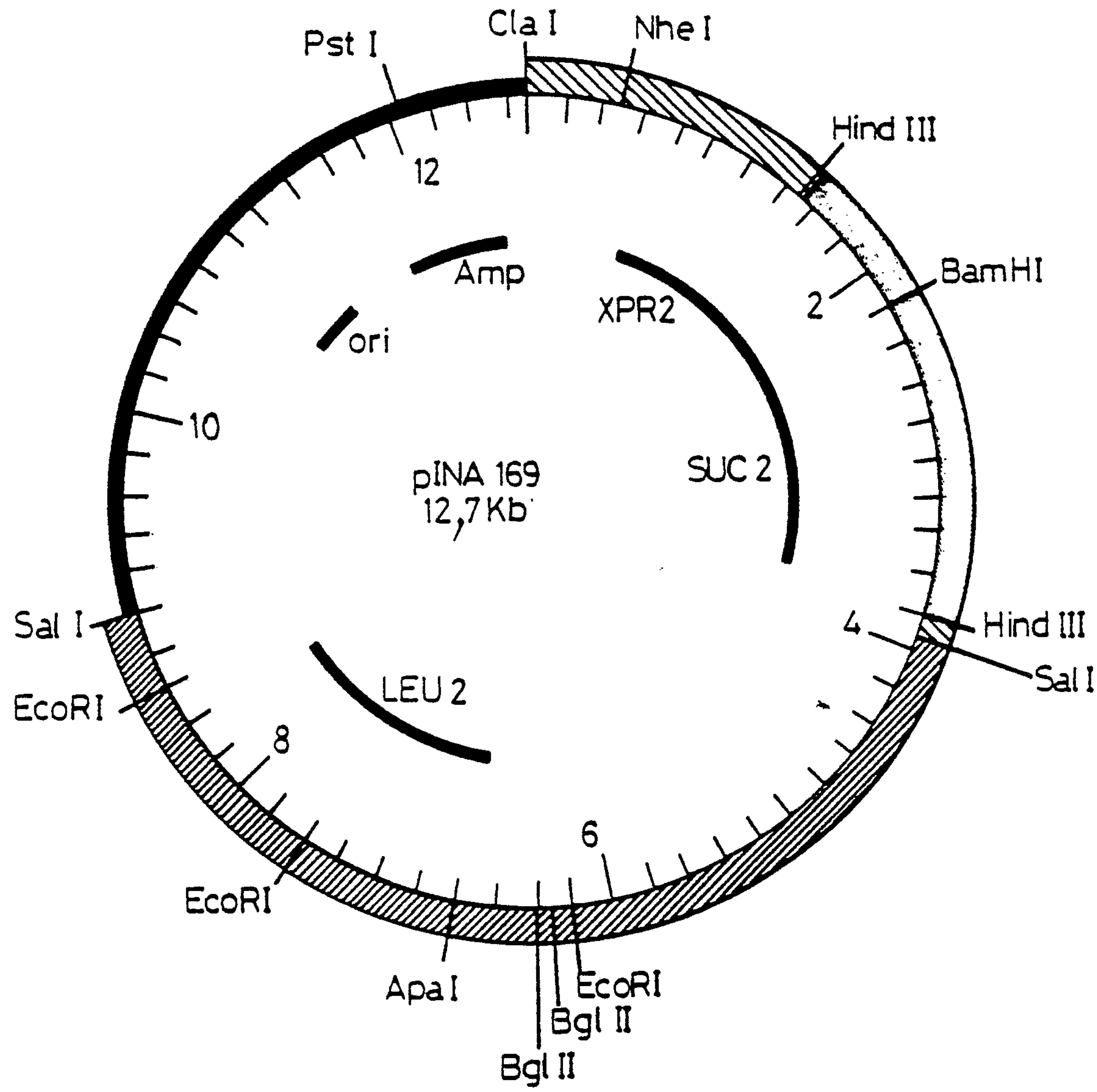
Cla I
Nhe I
Pst I
Hind III
BamHI
12
2
Amp
XPR2
ori
10
pINA 169
12,7 Kb
SUC 2
4
Hind III
Sal I
Sal I
LEU 2
EcoRI
8
6
EcoRI
Apa I
EcoRI
Bgl II
Bgl II

FIG.3

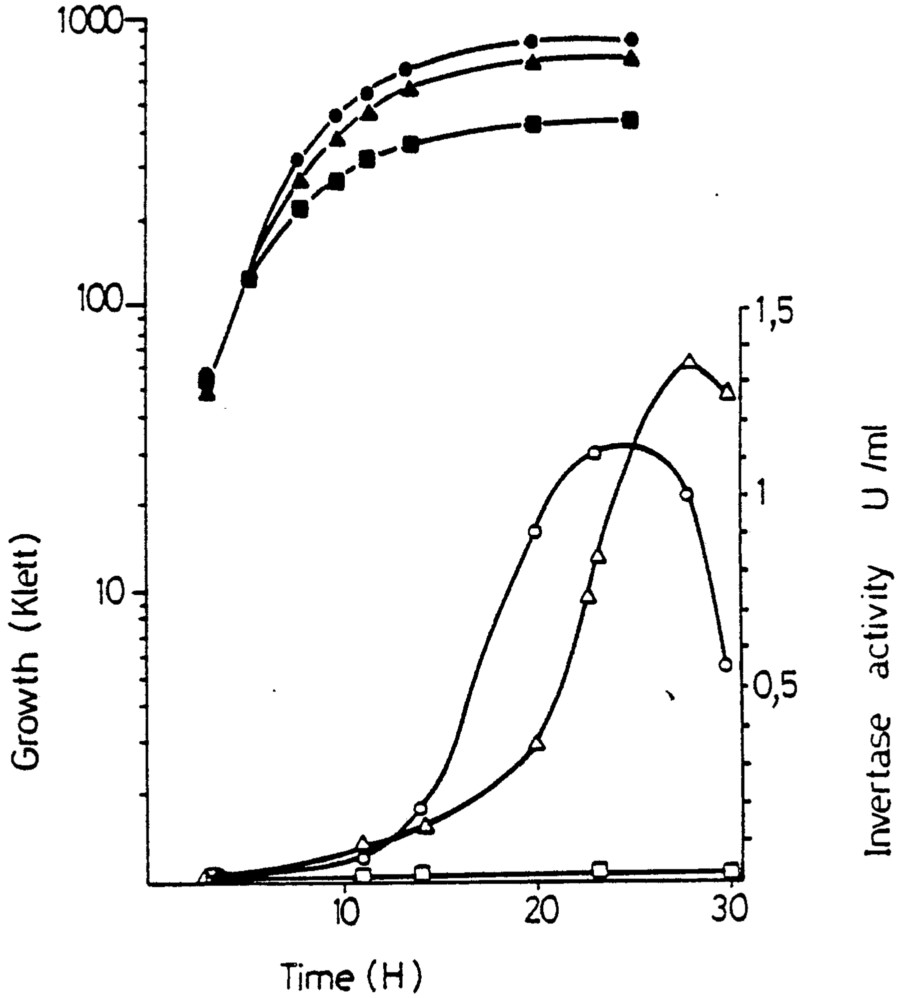
1000
100
10
Growth (Klett)
1,5
1
0,5
Invertase activity U /ml
10
20
30
Time (H)

FIG_4

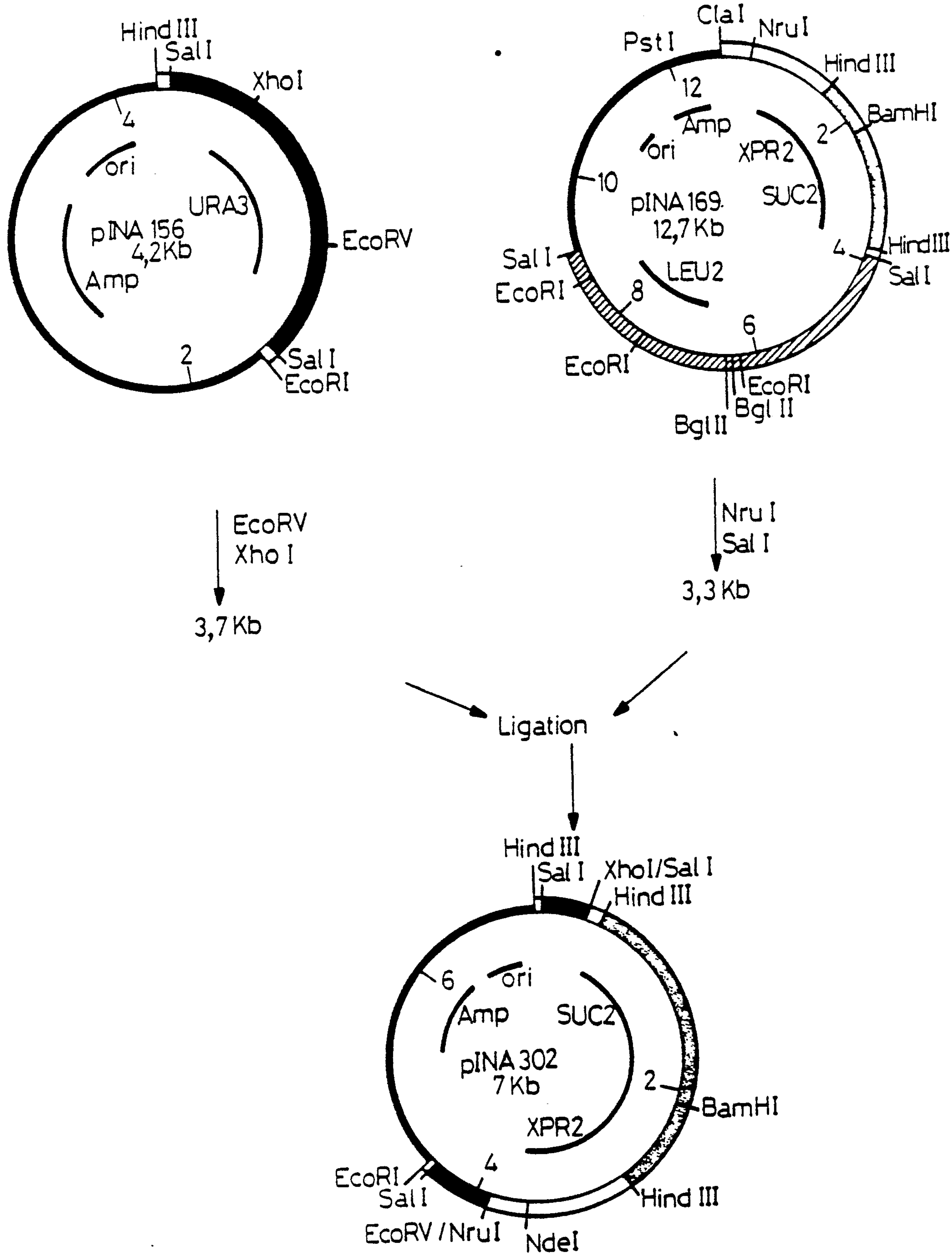
Hind III
Sal I
Xho I
4
ori
URA3
pINA 156
4,2Kb
Amp
EcoRV
2
Sal I
EcoRI
Cla I
Nru I
Pst I
12
Hind III
Amp
BamHI
2
ori
XPR2
10
pINA 169
12,7 Kb
SUC2
4
HindIII
Sal I
Sal I
EcoRI
LEU2
8
6
EcoRI
EcoRI
Bgl II
Bgl II
EcoRV
Xho I
3,7 Kb
Nru I
Sal I
3,3 Kb
Ligation
Hind III
Sal I
XhoI/Sal I
Hind III
6
ori
Amp
SUC2
pINA 302
7 Kb
2
BamHI
XPR2
4
EcoRI
Sal I
EcoRV / NruI
NdeI
Hind III

FIG.5

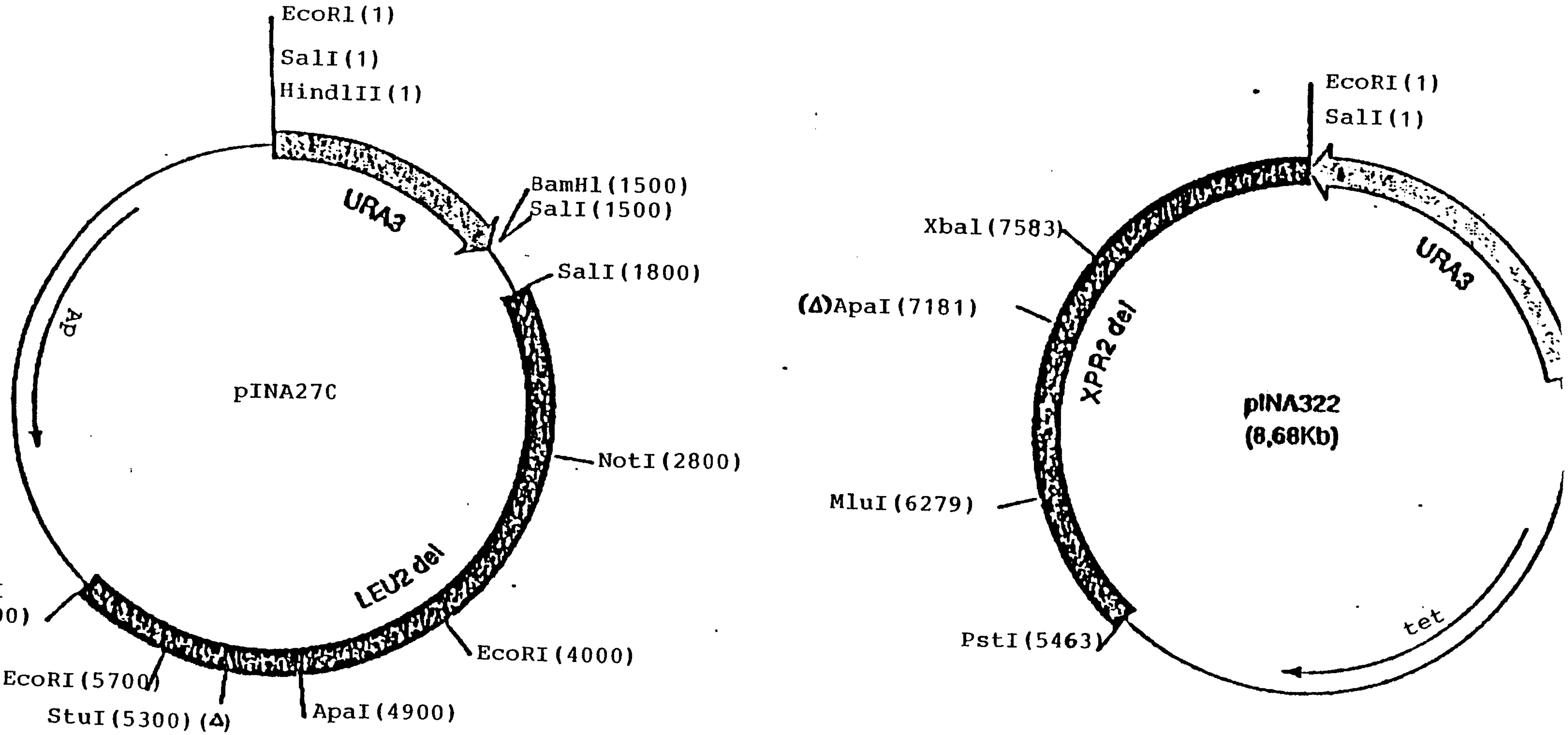


Restriction map of p' ısmids pINA270 and pIN'ʌ322.

European Patent Office

# EUROPEAN SEARCH REPORT

Application Number

EP 90 40 1503

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | JOURNAL OF BASIC MICROBIOLOGY, vol. 28, no. 3, 1988, pages 161-174; C. GAILLARDIN et al.: "Genetic engineering in Yarrowia lipolytica" --- | | C 12 N 15/62<br>C 12 N 15/65<br>C 12 N 15/81<br>C 12 N 1/16 //<br>(C 12 N 1/16<br>C 12 R 1:645) |
| A | EP-A-0 220 864 (PFIZER INC.) --- | | |
| A | EP-A-0 166 659 (INRA) --- | | |
| A | EP-A-0 261 534 (MILES LABORATORIES) --- | | |
| P,X | CURRENT GENETICS, vol. 16, July 1989, pages 253-260, Springer-Verlag, Berlin, DE; J.-M. NICAUD et al.: "Expression of invertase activity in Yarrowia lipolytica and its use as a selective marker" ----- | 1-19 | |
| | | | TECHNICAL FIELDS SEARCHED (Int. Cl.5) |
| | | | C 12 N |

The present search report has been drawn up for all claims

| Place of search | Date of completion of the search | Examiner |
|---|---|---|
| THE HAGUE | 20-08-1990 | VAN PUTTEN A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document